# EUROPEAN PATENT SPECIFICATION

(11) **EP 3 490 431 B1**
(45) Date of publication and mention of the grant of the patent: **07.10.2026**
(21) Application number: 16763561.4
(22) Date of filing: 29.07.2016
(51) Int. Cl.: A61B 3/032, A61B 3/00

(54) **MEASURING VISUAL ACUITY OF A CLIENT**
MESSUNG DER SEHSCHÄRFE EINES KUNDEN
MESURE DE L'ACUITÉ VISUELLE D'UN CLIENT

(43) Date of publication of application: 05.06.2019
(73) Proprietor: Medicontur Medical Engineering Ltd, 2072 Zsámbék (HU)
(72) Inventor: ERDEI, Gábor, 1118 Budapest (HU); FÜLEP, Csilla, 1027 Budapest (HU)
(74) Representative: Kacsuk, Zsófia
(86) International application number: PCT/HU2016/000050
(87) International publication number: WO 2018/020281

(56) References cited:
- CN-A- 101 313 843
- GB-A- 2 355 540
- US-B1- 6 379 007
- US-B1- 6 715 878
- US-B1- 9 277 857
- ANONYMOUS: "Correlation coefficient - Wikipedia", 23 July 2016 (2016-07-23), XP093339138, Retrieved from the Internet <URL:https://en.wikipedia.org/w/index.php?title=Correlation_coefficient&oldid=731149645>

## Description

### Technical Field

The invention relates in general to clinical visual acuity measurements. In particular, and not by way of limitation, the present invention is directed to a method, a system and a device for improving the measurement of visual acuity of a client.

### Background

Visual acuity is the most important ophthalmological quantity that describes the perceived resolving power of the human eye. Its measurement is based on symbol (letter, number or any type of character) recognition. However, recognition depends not only on the optical properties of the eye, but also on cognitive and motor abilities. Due to this complexity, visual acuity value is influenced by the mental state, fatigue and environmental factors. In clinical practice, conventional measurements are performed using visual acuity charts, or eye charts. The client's task is to correctly recognize the symbols, the size of which is decreasing from line to line. According to the "line assignment" scoring method, the visual acuity value corresponds to the line, where more than 50-60% of the symbols are recognized correctly (See 1. Duane T. (2006). Duane's Clinical Ophthalmology, Lippincott Williams & Wilkins, CD-ROM Edition. http://www.oculist.net/downaton502/prof/ebook/duanes/index.html), and 2. International Council of Ophthalmology, Visual Functions Committee (1988). Visual Acuity Measurement Standard, ICO 1984, Italian Journal of Ophthalmology, II/I 1988, pp 1 / 15.)
Its decimal metric is denoted by V, and is defined as $V = \frac{1}{α} ,$ where 5*α* is the angle of view of the smallest visible symbol in minutes of arc.

The measurement results are strongly influenced by many environmental parameters, such as the letter (symbol) style/contrast/color, the number of letters in a line, the illumination of the chart and the room, the testing distance, etc. There is no international standard for the parameter settings, but there are various traditional setups. For example, the ETDRS (Early Treatment of Diabetic Retinopathy Study) chart is used in lots of clinical studies and is considered as the US standard (Duane, 2006; International Council of Ophthalmology (ICO), 1984). It has a special layout with 5 letters in each line, where the spacing between the letters and between the lines equals the letter size. It is implemented with the so called SLOAN characters which have been devised specifically for visual acuity measurements to achieve approximately the same legibility for all the characters. The other settings are roughly uniform in most countries, but in some cases are very different.

The size of letters on current eye charts decreases by approximately 1/ 1.26× from line to line (i.e. 0.1 on logMAR scale), which limits accuracy in change for a rapid examination cycle. In contrast to clinical routine examinations, clinical research studies require higher accuracy and better repeatability. For this purpose there exist several scoring methods based on recording answers for individual letters, not lines ("single-letter" scoring). According to the most common practice, the logMAR value of the last line visible to the subject is increased by 0.02 per each letter mistaken, since there are traditionally 5 letters in a line (0.02 = 0.1/5) (Kaiser, P. K. (2009). Prospective Evaluation of Visual Acuity Assessment: a Comparison of Snellen Versus ETDRS Charts in Clinical Practice (an AOS Thesis), Transactions of the American Ophthalmological Society, 107:311-324). Though this certainly refines the recorded score, the obtained figure is hard to interpret since it does not correspond to the usual 60% probability threshold of line assignment scoring presented above.

In conventional single-letter scoring the examiner registers whether the client recognized the displayed letters correctly or not. This way the mere fact of recognition, or more precisely the recognition probability (P) is tested, therefore the answers are represented in a binary manner, by zeros and ones, corresponding to incorrect/correct answers (see for example documents CN 101 313 843 A, GB 2 355 540 A and US 9 277 857 B1). However, the situation is more complex in reality: in case of an incorrect answer it is not sure if the client does not see the specific letter at all. In other words, mixing up similar letters, such as "P" and "F", implies a better vision, than misidentifying totally different ones, such as "B" and "A".

Document US 2016089018 also relates to measuring visual acuity in which a system and a method are provided for measuring the visual acuity. The system comprises a computer or projector adapted to project a computer generated image of an optotype on a surface, e.g. a computer display screen or a screen on a wall, and a control unit. The computer or projector is adapted to project the optotypes with a steadily changing size as a continuum of images. The testing is carried out by changing the optotype size in both directions in order to compensate for the reaction time of the patient (client). For example, starting with a large optotype, the size is steadily decreased until the patient signals that he can no longer read the optotype. The test then continues starting from a small optotype and steadily increasing the size until the patient signals that he can read the optotype. The problem with respect to this related art is that it also does not take into account the similarities of different symbols.

We have set ourselves the objective with this invention to improve the visual acuity measurement.

### Summary of Invention

The present invention involves a method, a system and a computing device, which solves the aforementioned problems, as well as other problems that will become apparent from an understanding of the following description.

Accordingly, it is one aspect of the invention to provide a method for measuring visual acuity of a client as defined in claim 1.

In another aspect, the present invention is directed to a system which comprises a display device, an input device and a computing device as defined in claim 7.

In yet another aspect, the present invention refers to a computing device as defined in claim 12.

Present invention also relates to a computer program comprising instructions which, when executed by at least one processor of a computing device, causes the computing device to carry out the method according to the invention.

The invention also involves a storage containing the computer program as defined in claim 14.

In one of the advantageous embodiments, in order to quantify similarities between symbols (e.g. letters), the quantity of "optotype correlation" (OC) is introduced that is a pre-calculated value for each pair of symbols. Pre-calculation means that these values are calculated prior to the responses of the client. OC may be based on the appropriately modified Pearson's correlation values calculated across the symbols, and describes the specialties of these symbols on the mathematical basis of correlation. Possible values of OC cover the interval between -1 and +1, where larger values belong to better similarity. Specifically, +1 indicates a perfect match, 0 a random selection, and -1 means that the two symbols are just the contrary of each other. During a visual acuity measurement the pre-calculated OC values are recorded for each symbol in the same size, according to the responses of the client. The client's visual performance is quantified by a value of "rate of recognition" (RR) that is the average of the OC values the client produced for a set of symbols of the same size.

The most important advantage of the invention is that it decreases the uncertainty of visual acuity measurements.

### Brief Description of Drawings

For a more complete understanding of the invention, reference is made to the following detailed description taken in conjunction with the accompanying drawings wherein:
Fig. 1 is an example of correlating two symbols;
Fig. 2 is an example of numerical values of optotype correlation for the first five letters of the English alphabet;
Fig. 3 depicts an example of the relation between rate of recognition (RR) and the reciprocal angle of view (v);
Fig. 4 shows a combined picture of an example of the method, the system and the calculating device for measuring visual acuity;
Fig. 5 is an example of the measuring method;
Fig. 6 is an example of the computing device.

### Detailed Description of Embodiments

Quantifying similarities between symbols may be carried out on the basis of correlation calculation. This metric is called "optotype correlation" (OC). The term "optotype" stands for symbols, characters or numbers in the prior art. OC must not depend on how the client exactly sees symbols; instead it should compare symbols in their original form in order to avoid client-specific artefacts. In addition, the OC value cannot be affected by the symbol size either, only the shape of the symbol should be considered in its definition. For this purpose, pre-calculation of the OC is carried out on the non-distorted, high-resolution black-and-white images of a set of symbols, e.g. on the images of capital letters of the English alphabet, where the images are represented as two-dimensional matrices. The mathematical function that has been developed specifically for image comparison is called Pearson's correlation, which characterizes similarity of two pictures by a single scalar number *ρ*(*p*, *q*), according to: $ρ \left(p, q\right) = \frac{{\sum }_{x , y} \left[f\left(x, y\right)-\overline{f}\right] . \left[g\left(x-p,y-q\right)-\overline{g}\right]}{\sqrt{{\sum }_{x , y} {\left[f\left(x, y\right)-\overline{f}\right]}^{2} ⋅ {\sum }_{x , y} {\left[g\left(x-p,y-q\right)-\overline{g}\right]}^{2}}} .$ In the above equation *f*(*x*, *y*) and *g*(*x*, *y*) are the matrices of the two symbols to be compared, *p* and *q* refer to the relative lateral shift between the matrices and *f̅* indicates the mean value of *f*(*x*, *y*). Pixel coordinates are denoted by *x-y* and *p-q.* The matrices of the symbols are binary, square matrices, in which a character is covered by 150×150 elements (i.e. pixels). The cells of the black symbol are zeros, while the cells of the white background are represented by ones. Each symbol is surrounded by an additional 150 pixel-wide white border around the symbol in order to avoid numerical artefacts during the pre-calculations. Possible values of *ρ* are between -1 and + 1, where + 1 indicates identical matrices, larger values belong to more related matrices, 0 to a random selection, and -1 means that the two matrices are just the contrary of each other. The value of *ρ* significantly depends on how the two matrices are shifted relative to each other. For quantifying optotype correlation we always selected the case in which the correlation value is at its maximum: $ρ = {max}_{p , q} \left\{ρ\left(p, q\right)\right\} .$

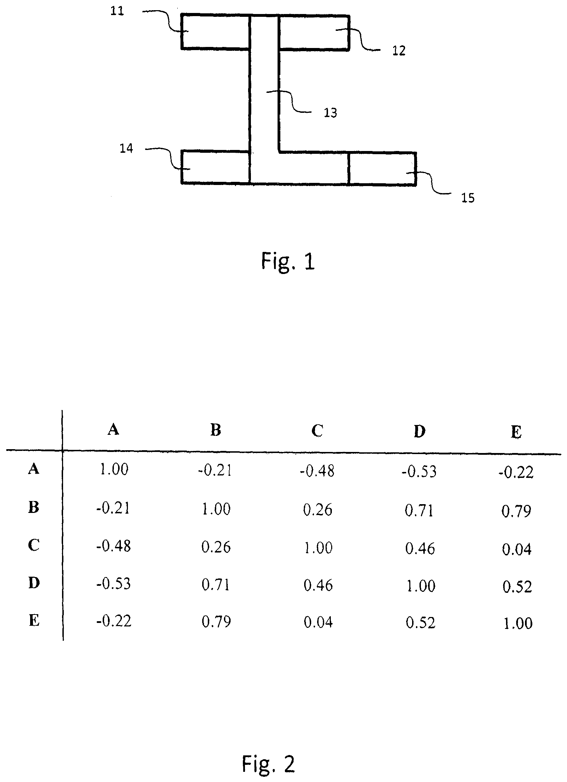

For an example see Fig. 1, where letters "L" and "I" are in maximum correlation position. Area 11, 12, 13, 14 belong to letter "I" and area 13, 15 belong to letter "L". In order that the distribution of correlation values for random responses of the client become consistent with the usual indication of false answers by zeros, the expected value of correlations is modified in case of incorrect recognitions to zero. This is how we obtain OC: $OC ≡ \frac{ρ - \overline{ρ}}{1 - \overline{ρ}} ,$ where *ρ̅* indicates the expected value of the Pearson's correlation distribution without the unity values. The above linear transform ensures that the expected value of misidentifications is zero, and the correct recognitions are represented as unity.

The numerical values of OC for the first five letters of the English alphabet, in case of SLOAN characters, are shown in Fig. 2. The OC matrix, for all 26 letters of the English alphabet, is symmetric, which means that the similarity operation is commutative for its variables. For identical letters, those in the main diagonal of the matrix, the values are unity. Moreover, for more similar letters, such as "B" and "E", the optotype correlation is larger (0.79), than for less similar letters, such as "A" and "B" (-0.21). It follows from the equation above, that in case of small letters (when the client does not see the letters at all) the average optotype correlation obtained at a given letter size equals the recognition probability. If we use all the twenty six letters of the English alphabet during the examination, the value of recognition probability is P = 1/26 ≈ 0.04. The results are in good agreement with this theoretical expectation: the average value of the whole OC matrix, with the unity values, is 0.04. At the same time, in case of large letters, when the client sees every detail of the characters, the expected value of both the optotype correlation and the recognition probability is 1.

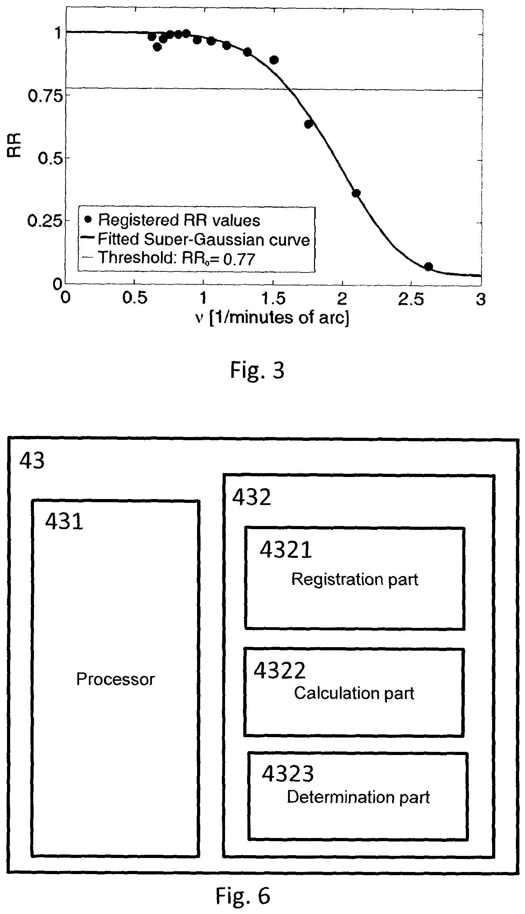

According to the above discussion, the average value of the OC is directly comparable to recognition probability (P), but provides more information about vision. For this reason a new metric is proposed to quantify visual acuity at a given symbol size called as the rate of recognition (RR): $RR ≡ \overline{OC} ,$ which is the average of OC values at a given symbol size. It must be noted that in the intermediate region, when the client sees some blur from the symbols, the RR is always somewhat larger than recognition probability (P). In Fig. 3, the RR results of a typical measurement is presented for a clear understanding of the relation between RR and the reciprocal angle of view (ν) $ν ≡ \frac{1}{α}$, in which *α* is proportional to the size of the symbol. The measured points in Fig. 3 tendentiously follow a trace that can be apparently fitted by a smooth curve. Since presently there is no theoretical explanation for the shape of this curve, an analytical form is selected, i.e. a function is calculated by fitting to the RR values of each symbol size. The main aspect was to provide a robust fit, thus the so-called Super-Gaussian (SG) function was used.
Visual acuity (V) can be determined precisely for a given client from the Super-Gaussian curve fitted to his/her registered RR values: the measured visual acuity corresponds to the specific symbol size (ν₀) at which the RR equals (or drops below) a given threshold (RR₀). This can be mathematically expressed as ${RR}_{SG} \left({ν}_{0}\right) = {RR}_{0} ⇒ V ≡ {ν}_{0} .$

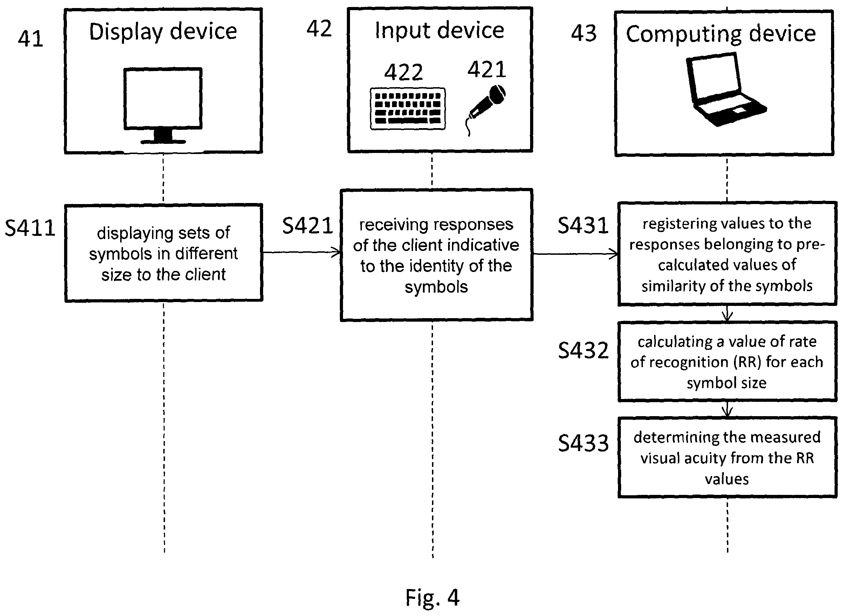

In Fig. 4, a combined diagram is provided to demonstrate an example of the method, the system and the computing unit for measuring visual acuity. On a display device (41), sets of symbols in different size are displayed (S411) to the client. Symbols can be characters, letters or optotypes of different size.

An input device 42 receives S421 responses of the client indicative to the identity of the symbols. Responses can be voice or tactile reactions about the identity of the symbols entered to the input device 42, so the input device 42 can be e.g. a microphone with voice recognition module 421 to receive an oral answer from the client or can be a tactile reaction recognition module 422, such as a keyboard, that are operative to receive responses of the client indicative to the identity of the symbols and sending the information of the response to the computing device 43. The separation of the display device 41, the input device 42 and the computing device 43 is based on the function they carry out and not on the physical entities in which they are implemented. E.g., the display device 41, the input device 42 and the computing device 43 can be implemented in a single notebook having a keyboard, a monitor comprised in or connected to the computing device of the notebook and a processor with memory capable of controlling the monitor for generating the images of the symbols. In another implementation the display device 41 can be a screen on a wall displaying the images of symbols projected by a projector. The projector can be also under control of the notebook but can be operated by a different entity.

In the computing device 43, values to the responses belonging to pre-calculated values of similarity of the symbols are registered S431. In this step, a value of "1" is registered if the response of the client is true, i.e. the identity of the symbol displayed on the display device 41 is identical to the response. On the other way, when the response of the client is false, a pre-calculated value is registered. This pre-calculated value is identical to the value calculated for the similarity of pair of symbols, shown in Fig. 2. In an advantageous embodiment the similarity can be pre-calculated as optotype correlation (OC). E.g., the displayed symbol was "C" and the client response was "C". In this case, the registered value is "1". If the displayed symbol was "C", but the client response was "D", then the registered value is "0.46", which is the value in the field of column "C" and row "D". The next step is the calculation S432 of the rate of recognition (RR) value for each symbol size. RR may be calculated as the average of the registered values for each symbol size.

In the next step, the measured visual acuity is determined S433 from the RR values. Determination may include steps of calculating a function fitting to the RR values of each symbol size and defining visual acuity belonging to a RR threshold (RR₀).

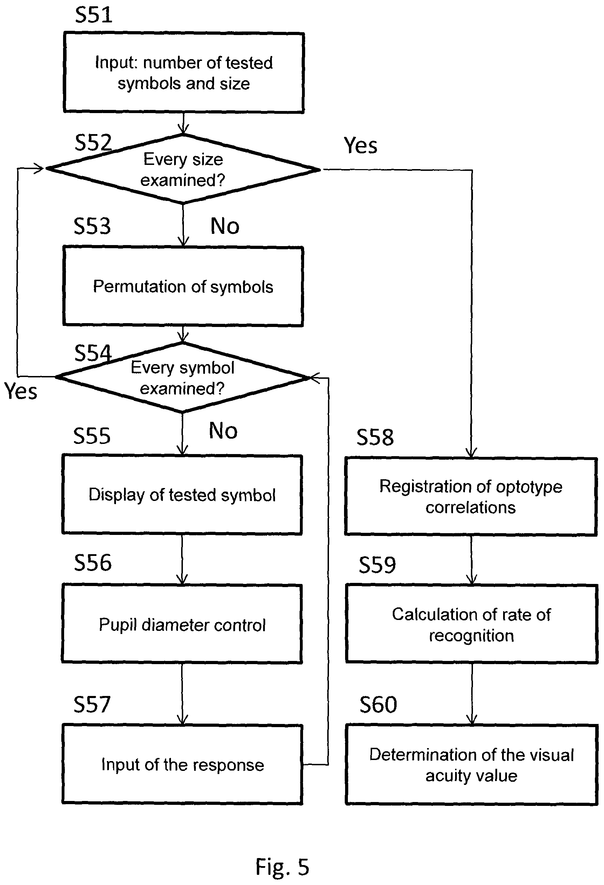

In order to demonstrate the usefulness of the visual acuity measurement method of this invention, and to provide data for the RR₀ calibration process, a special measurement setup was implemented, in which the environmental conditions were kept under tight control. Symbols were presented to the client one by one on a computer screen (LCD monitor) implementing the function of the display device. The test distance has been selected to be large enough to ensure accommodation-free measurements. The depth of field of the human eye is 1/4 diopter, which means that the test distance has to be larger than 4 meters. An in-plane-switching LCD monitor was used, with a pixel pitch of 0.265 millimeter. So that symbols were displayed with sufficiently large resolution, the test distance was set to 9.5 meters. This relatively large distance allows for a denser sampling of the visual acuity scale (ΔlogMAR ≈ 0.05) than attainable in clinical measurements, which further decreases the error of the results. For the measurement each symbol size (14 in total) was used for which the stroke width of the symbols was an integral multiple of the pixel pitch. Since the human pupil is wider under mesoscopic than photopic conditions, we carried out our measurements in a darkened room, with an illuminance of around 10 lux (i.e. 3.2 cd/m² average luminance). In such an environment the refractive errors (chromatic and higher order monochromatic aberrations) have more significant effect on visual acuity. The luminance of the monitor was 90 cd/m²-s that fulfills the ICO standard (min. 80 cd/m²). An example of this method is shown in Fig. 5.

The most important input parameters S51 of the program are the symbol sizes to be displayed and the number of the tested symbols at a size. One of the main advantages of the PC-based setup is that we can perform customized measurements, i.e. we can fit the test parameters to the currently examined client. In addition, only one test distance is sufficient to examine clients in a wide visual acuity range, which ensures easy implementation as well as accurate and reliable results. During the measurement the algorithm runs over the symbol sizes S52 and symbol types S54, and permutes S53 the symbols in each size. This way, the client cannot learn the sequence of the symbols by heart. The algorithm outputs S55 the symbols on the monitor and waits for the response. After having the response, the displayed-identified symbol pair is saved for further analysis. The next test symbol is always displayed only after the response has been given S57. The symbols are shown on a permanent white background, one by one, so that crowding has no influence on the measurement. The "one at a time" displaying method makes it possible to examine all symbols, e.g., all the twenty six capital letters of the English alphabet in each letter size, instead of the five characters printed in a line on a visual acuity chart according to the prior art. Due to the increased number of tested symbols this setup provides more information than clinical measurements, which statistically decreases the error of the results. Furthermore, the fact that e.g. all the twenty six letters of the English alphabet are examined in each letter size ensures that the client has to perform exactly the same task in each size, which provides reliable acuity scoring. For fourteen letter sizes (covering the normal and supernormal visual acuity range of eye charts, i.e. from 0.2 to -0.4 logMAR value) and twenty six optotypes in a row the measurement takes approximately half an hour.

During the measurements, as in clinical ones, the client watched the monitor with one eye, while the other was covered with a transparent but opaque shield. In other words, visual acuity is determined separately for the two eyes. Since the pupil size significantly influences visual acuity, we continuously controlled S56 the pupil diameter during the visual acuity test with a digital camera. Responses were registered S58 with the optotype correlation and rate of recognition was calculated S59. Based on the RRs, visual acuity is determined S60.

In Fig. 6, an example of the computing device 43 is illustrated. The computing device 43 receives information from an input device 42 for computing visual acuity of a client. In this embodiment the computing device 43 comprises a processor 431 and a storage 432. Said storage 432 containing instructions executable by said processor 431 whereby said computing device 43 is operative to register values S431 to the responses belonging to pre-calculated values of similarity of the symbols, to calculate S432 a value of rate of recognition, RR, for each symbol size and to determine S433 the measured visual acuity from the RR values. The computing device 43 is also operative to calculate S432 RR as the average of the registered values for each symbol size and to determine S433 the measured visual acuity from the RR values. Determining S433 may comprise steps of calculating a function fitting to the RR values of each symbol size and defining visual acuity belonging to a RR threshold, RR₀.

The computing device 43 is operated by a computer program comprising instructions which, when executed by at least one processor 431 causes the computing device 43 to carry out the steps of registering S431 values to the responses belonging to pre-calculated values of similarity of the symbols, calculating S432 a RR value, for each symbol size, and determining S433 the measured visual acuity from the RR values.

The computing device 43 comprises a storage 432 containing at least a registration part 4321, a calculation part 4322 and a determination part 4323 to carry out the steps of registering S431 values to the responses belonging to pre-calculated values of similarity of the symbols, calculating S432 a RR value, for each symbol size and determining S433 the measured visual acuity from the RR values, respectively.

Although preferred embodiments of the present invention have been illustrated in the accompanying drawings and described in the foregoing detailed description, it is understood that the invention is not limited to embodiments disclosed but is capable of numerous rearrangements, modifications, and substitutions for visual acuity measurements without departing from the invention, as realized and defined by the following claims.

## Claims

1. A method for measuring visual acuity of a client, said method comprising the steps of
- on a display device (41), displaying (S411) sets of symbols in different size to the client,
- in an input device (42), receiving (S421) responses of the client indicative of the identity of the symbols,
- in a computing device (43),
a) registering (S431) a pre-calculated value of similarity for each response the pre-calculated value of similarity being a correlation value calculated for a pair of symbols consisting of the displayed symbol and the identified symbol indicated in the response,
b) calculating (S432) a value of rate of recognition, RR, as an average of the registered values for each symbol size,
c) determining (S433) the measured visual acuity from the RR values.

2. The method of claim 1, in which the symbols are characters, letters or optotypes of different size.

3. The method of claim 1, in which responses are voice or tactile reactions about the identity of the symbols entered to the input device (42).

4. The method of claim 1, in which correlation values are optotype correlations, OC, and calculated by $OC ≡ \frac{ρ - \overline{ρ}}{1 - \overline{ρ}}$, where, *ρ* is the Pearson's correlation distribution of all symbols, and *ρ̅* indicates the expected value of the Pearson's correlation distribution without the unity values.

5. The method of claim 1, in which determining the measured visual acuity from the RR values comprises
- calculating a function fitting to the RR values of each symbol size and
- defining visual acuity belonging to a RR threshold, RR₀.

6. The method of claim 5, in which the function fitting to the RR values is a Super-Gaussian (SG) function.

7. A system for measuring visual acuity of a client, comprising a display device (41), an input device (42) and a computing device (43), wherein
- the display device (41) is adapted to displaying sets of symbols in different size to the client;
- the input device (42) is adapted to receiving responses of the client indicative of the identity of the symbols, and- the computing device (43) is adapted to
a) registering a pre-calculated value of similarity for each response, the pre-calculated value of similarity being a correlation value calculated for a pair of symbols consisting of the displayed symbol and the identified symbol indicated in the response,
b) calculating a value of rate of recognition, RR, from the registered values for each symbol size, and
c) determining the measured visual acuity from the RR values.

8. The system of claim 7, in which the computing device (43) is capable of controlling the display device for generating the images of the symbols.

9. The system of claim 7, in which the display device (41) is a screen displaying the images of symbols projected by a projector.

10. The system of claim 7, in which the display device (41) is a monitor that is comprised in or connected to the computing device.

11. The system of claim 7, in which the input device (42) includes a voice recognition module (421) or a tactile reaction recognition module (422) that are operative to receive responses of the client indicative to the identity of the symbols.

12. A computing device (43) receiving information from an input device (42) for computing visual acuity of a client, said computing device (43) comprising a processor (431) and a storage (432), said storage (432) containing instructions executable by said processor (431) whereby said computing device (43) is operative to carry out the method of any one of claims 1 to 6.

13. A computer program comprising instructions which, when executed by at least one processor of a computing device, causes the computing device to carry out the method of any of claims 1-5.

14. A storage (432) containing the computer program according to claim 13, comprising at least a registration part (4321), a calculation part (4322) and a determination part (4323).

## Patentansprüche

1. Verfahren zum Messen der Sehschärfe eines Klienten, wobei das Verfahren die Schritte umfasst:
- auf einer Anzeigevorrichtung (41), Anzeigen (S411) von Sätzen von Symbolen unterschiedlicher Größe für den Klienten,
- in einer Eingabevorrichtung (42), Empfangen (S421) von Antworten des Klienten, welche die Identität der Symbole angeben,
- in einer Rechenvorrichtung (43)
a) Registrieren (S431) eines vorab berechneten Ähnlichkeitswertes für jede Antwort, wobei der vorab berechnete Ähnlichkeitswert ein Korrelationswert ist, der für ein Paar von Symbolen berechnet wird, das aus dem angezeigten Symbol und dem in der Antwort angegebenen identifizierten Symbol besteht,
b) Berechnen (S432) eines Wertes der Erkennungsrate, RR, als Durchschnitt der registrierten Werte für jede Symbolgröße,
c) Bestimmen (S433) der gemessenen Sehschärfe aus den RR-Werten.

2. Verfahren nach Anspruch 1, wobei die Symbole Zeichen, Buchstaben oder Optotypen unterschiedlicher Größe sind.

3. Verfahren nach Anspruch 1, wobei die Antworten sprachliche oder taktile Reaktionen bezüglich der Identität der Symbole sind, die in die Eingabevorrichtung (42) eingegeben werden.

4. Verfahren nach Anspruch 1, wobei die Korrelationswerte Optotypenkorrelationen, OC, sind und gemäß $OC ≡ \frac{ρ - \overline{ρ}}{1 - \overline{ρ}}$ berechnet werden, wobei *ρ* die Pearson-Korrelationsverteilung aller Symbole ist und *ρ̅* den Erwartungswert der Pearson-Korrelationsverteilung ohne die Einheitswerte angibt.

5. Verfahren nach Anspruch 1, wobei das Bestimmen der gemessenen Sehschärfe aus den RR-Werten umfasst:
- Berechnen einer an die RR-Werte jeder Symbolgröße angepassten Funktion und
- Definieren einer zu einem RR-Schwellenwert, RR₀, gehörenden Sehschärfe.

6. Verfahren nach Anspruch 5, wobei die an die RR-Werte angepasste Funktion eine Super-Gauß-Funktion (SG) ist.

7. System zum Messen der Sehschärfe eines Klienten, umfassend eine Anzeigevorrichtung (41), eine Eingabevorrichtung (42) und eine Rechenvorrichtung (43), wobei
- die Anzeigevorrichtung (41) dazu eingerichtet ist, Sätze von Symbolen unterschiedlicher Größe für den Klienten anzuzeigen;
- die Eingabevorrichtung (42) dazu eingerichtet ist, Antworten des Klienten zu empfangen, welche die Identität der Symbole angeben; und
- die Rechenvorrichtung (43) dazu eingerichtet ist
a) einen vorab berechneten Ähnlichkeitswert für jede Antwort zu registrieren, wobei der vorab berechnete Ähnlichkeitswert ein Korrelationswert ist, der für ein Paar von Symbolen berechnet wird, das aus dem angezeigten Symbol und dem in der Antwort angegebenen identifizierten Symbol besteht,
b) einen Wert der Erkennungsrate, RR, aus den registrierten Werten für jede Symbolgröße zu berechnen, und
c) die gemessene Sehschärfe aus den RR-Werten zu bestimmen.

8. System nach Anspruch 7, wobei die Rechenvorrichtung (43) dazu eingerichtet ist, die Anzeigevorrichtung zur Erzeugung der Bilder der Symbole zu steuern.

9. System nach Anspruch 7, wobei die Anzeigevorrichtung (41) ein Bildschirm ist, der die von einem Projektor projizierten Bilder von Symbolen anzeigt.

10. System nach Anspruch 7, wobei die Anzeigevorrichtung (41) ein Monitor ist, der Bestandteil der Rechenvorrichtung ist oder mit der Rechenvorrichtung verbunden ist.

11. System nach Anspruch 7, wobei die Eingabevorrichtung (42) ein Spracherkennungsmodul (421) oder ein Modul zur Erkennung taktiler Reaktionen (422) umfasst, die dazu eingerichtet sind, Antworten des Klienten zu empfangen, welche die Identität der Symbole angeben.

12. Rechenvorrichtung (43), die Informationen von einer Eingabevorrichtung (42) zum Berechnen der Sehschärfe eines Klienten empfängt, wobei die Rechenvorrichtung (43) einen Prozessor (431) und einen Speicher (432) umfasst, wobei der Speicher (432) Anweisungen enthält, die von dem Prozessor (431) ausführbar sind, wodurch die Rechenvorrichtung (43) dazu eingerichtet ist, das Verfahren nach einem der Ansprüche 1 bis 6 auszuführen.

13. Computerprogramm, umfassend Anweisungen, die bei Ausführung durch mindestens einen Prozessor einer Rechenvorrichtung bewirken, dass die Rechenvorrichtung das Verfahren nach einem der Ansprüche 1 bis 5 ausführt.

14. Speicher (432), der das Computerprogramm nach Anspruch 13 enthält und mindestens einen Registrierungsteil (4321), einen Berechnungsteil (4322) und einen Bestimmungsteil (4323) umfasst.

## Revendications

1. Procédé de mesure de l'acuité visuelle d'un client, ledit procédé comprenant les étapes consistant à :
- sur un dispositif d'affichage (41), afficher (S411) des ensembles de symboles de tailles différentes au client,
- dans un dispositif d'entrée (42), recevoir (S421) des réponses du client indiquant l'identité des symboles,
- dans un dispositif de calcul (43) :
a) enregistrer (S431) une valeur de similarité précalculée pour chaque réponse, la valeur de similarité précalculée étant une valeur de corrélation calculée pour une paire de symboles constituée du symbole affiché et du symbole identifié indiqué dans la réponse,
b) calculer (S432) une valeur du taux de reconnaissance, RR, en tant que moyenne des valeurs enregistrées pour chaque taille de symbole,
c) déterminer (S433) l'acuité visuelle mesurée à partir des valeurs RR.

2. Procédé selon la revendication 1, dans lequel les symboles sont des caractères, des lettres ou des optotypes de tailles différentes.

3. Procédé selon la revendication 1, dans lequel les réponses sont des réactions vocales ou tactiles concernant l'identité des symboles, introduites dans le dispositif d'entrée (42).

4. Procédé selon la revendication 1, dans lequel les valeurs de corrélation sont des corrélations d'optotypes, OC, et sont calculées selon $OC ≡ \frac{ρ - \overline{ρ}}{1 - \overline{ρ}}$ où *ρ* est la distribution de corrélation de Pearson de tous les symboles, et *ρ̅* indique la valeur espérée de la distribution de corrélation de Pearson sans les valeurs égales à l'unité.

5. Procédé selon la revendication 1, dans lequel la détermination de l'acuité visuelle mesurée à partir des valeurs RR comprend :
- le calcul d'une fonction ajustée aux valeurs RR de chaque taille de symbole, et
- la définition d'une acuité visuelle correspondant à un seuil RR, RR₀.

6. Procédé selon la revendication 5, dans lequel la fonction ajustée aux valeurs RR est une fonction super-gaussienne (SG).

7. Système de mesure de l'acuité visuelle d'un client, comprenant un dispositif d'affichage (41), un dispositif d'entrée (42) et un dispositif de calcul (43), dans lequel :
- le dispositif d'affichage (41) est adapté pour afficher des ensembles de symboles de tailles différentes au client ;
- le dispositif d'entrée (42) est adapté pour recevoir des réponses du client indiquant l'identité des symboles ; et
- le dispositif de calcul (43) est adapté pour :
a) enregistrer une valeur de similarité précalculée pour chaque réponse, la valeur de similarité précalculée étant une valeur de corrélation calculée pour une paire de symboles constituée du symbole affiché et du symbole identifié indiqué dans la réponse,
b) calculer une valeur du taux de reconnaissance, RR, à partir des valeurs enregistrées pour chaque taille de symbole, et
c) déterminer l'acuité visuelle mesurée à partir des valeurs RR.

8. Système selon la revendication 7, dans lequel le dispositif de calcul (43) est apte à commander le dispositif d'affichage pour générer les images des symboles.

9. Système selon la revendication 7, dans lequel le dispositif d'affichage (41) est un écran affichant les images de symboles projetées par un projecteur.

10. Système selon la revendication 7, dans lequel le dispositif d'affichage (41) est un moniteur qui est compris dans le dispositif de calcul ou connecté au dispositif de calcul.

11. Système selon la revendication 7, dans lequel le dispositif d'entrée (42) comprend un module de reconnaissance vocale (421) ou un module de reconnaissance de réactions tactiles (422), qui sont aptes à recevoir des réponses du client indiquant l'identité des symboles.

12. Dispositif de calcul (43) recevant des informations provenant d'un dispositif d'entrée (42) pour calculer l'acuité visuelle d'un client, ledit dispositif de calcul (43) comprenant un processeur (431) et une mémoire (432), ladite mémoire (432) contenant des instructions exécutables par ledit processeur (431), grâce auxquelles ledit dispositif de calcul (43) est apte à mettre en œuvre le procédé selon l'une quelconque des revendications 1 à 6.

13. Programme d'ordinateur comprenant des instructions qui, lorsqu'elles sont exécutées par au moins un processeur d'un dispositif de calcul, amènent le dispositif de calcul à mettre en œuvre le procédé selon l'une quelconque des revendications 1 à 5.

14. Mémoire (432) contenant le programme d'ordinateur selon la revendication 13, comprenant au moins une partie d'enregistrement (4321), une partie de calcul (4322) et une partie de détermination (4323).
